# EUROPEAN PATENT SPECIFICATION

(11) **EP 1 545 694 B1**
(45) Date of publication and mention of the grant of the patent: **25.02.2009**
(21) Application number: 03770472.3
(22) Date of filing: 25.09.2003
(51) Int. Cl.: A61N 1/05

(54) **PROCESS FOR FORMING AN IMPLANTABLE MEDICAL DEVICE LEAD CONDUCTOR**
HERSTELLUNGSVERFAHREN EINES IMPLANTIERBAREN MEDIZINISCHEN LEITERS
PROCEDE DE FORMATION D'UN CONDUCTEUR D'ELECTRODE DE DISPOSITIF MEDICAL IMPLANTABLE

(30) Priority: 30.09.2002 US 261014
(43) Date of publication of application: 29.06.2005
(60) Divisional application: 09000298.1
(73) Proprietor: MEDTRONIC, INC., Minneapolis MN 55432-5604 (US)
(72) Inventor: LESSAR, Joseph, F., Coon Rapids, MN 55432 (US); MARTINEZ, Gonzalo, Mendota Heights, MN 55118 (US); BOSER, Gregory, A., Richfield, MN 55423 (US); WISER, Jeff, A., Coon Rapids, MN 55126 (US); MCINTYRE, Peter, B., Mounds View, MN 55112 (US); MAYER, David, W., Bloomington, MN 55437 (US); BAKER, Paul, D., Oakdale, MN 55128 (US)
(74) Representative: Hughes, Andrea Michelle
(86) International application number: PCT/US2003/030375
(87) International publication number: WO 2004/030751

(56) References cited:
- EP-A- 1 005 879
- EP-A- 1 070 515
- US-A- 4 711 027
- US-A- 5 330 521
- US-A- 5 466 252
- US-A- 5 849 031
- US-B1- 6 253 111

## Description

The present invention relates generally to implantable medical devices for delivering electrical stimulation to a target area of a patient, and in particular, the present invention relates to a conductor material having integral oxide insulation utilized in implantable medical device leads of implantable medical devices.

A wide assortment of implantable medical devices (IMDs) are presently known and in commercial use. Such devices include cardiac pacemakers, cardiac defibrillators, cardioverters, neurostimulators, and other devices for delivering electrical signals to a portion of the body and/or receiving signals from the body. Pacemakers, for example, are designed to operate so as to deliver appropriately timed electrical stimulation signals when needed, in order to cause the myocardium to contract or beat, and to sense naturally occurring conduction signals in the patient's heart.

Devices such as pacemakers, whether implantable or temporary external type devices, are part of a system for interacting with the patient. In addition to the pacemaker device, which typically has some form of pulse generator, a pacing system includes one or more leads for delivering generated stimulation pulses to the heart and for sensing cardiac signals and delivering sensed signals from the heart back to the pacemaker. As is known, pacemakers can operate in either a unipolar or bipolar mode, and can pace the atria or the ventricles. Unipolar pacing requires a lead having only one distal electrode for positioning in the heart, and utilizes the case, or housing of the implanted device as the other electrode for the pacing and sensing operations. For bipolar pacing and sensing, the lead typically has two electrodes, a tip electrode disposed at the distal end of the lead, and a ring electrode spaced somewhat back from the distal end. Each electrode is electrically coupled to a conductive cable or coil, which carries the stimulating current or sensed cardiac signals between the electrodes and the implanted device via a connector electrically coupled to the device.

In the field of cardiac stimulation and monitoring, endocardial leads are placed through a transvenous route to position one or more sensing and/or stimulation electrodes in a desired location within a heart chamber or interconnecting vasculature. During this type of procedure, a lead is passed through the subclavian, jugular, or cephalic vein, into the superior vena cava, and finally into a chamber of the heart or the associated vascular system. An active or passive fixation mechanism at the distal end of the endocardial lead may be deployed to maintain the distal end of the lead at a desired location.

Routing an endocardial lead along a desired path to a target implant site can be difficult and is dependent upon the physical characteristics of the lead. At the same time, as will be readily appreciated by those skilled in the art, it is highly desirable that the implantable medical lead insulation possess high dielelectric properties, and exhibit durable and bio-stable mechanical and electrical properties, predictable and consistent flexibility, sufficient flex fatigue resistance, wear/abrasion resistance, and minimal thickness.

In order to perform reliably, cardiac pacing leads need to be positioned and secured at a targeted cardiac tissue site in a stable manner. One common mechanism for securing an electrode position is the use of a rotatable fixation helix. The helix exits the distal end of the lead and can be screwed into the body tissue. The helix itself may serve as an electrode or it may serve as an anchoring mechanism to locate an electrode mounted to the lead body adjacent a targeted tissue site. The fixation helix may be coupled to a drive shaft that is further connected to a coiled conductor that extends through the lead body as generally described in U.S. Pat. No. 4,106,512 to Bisping et al. A physician rotates the coiled conductor at a proximal end to cause rotation of the fixation helix via the drive shaft. As the helix is rotated in one direction, the helix is secured in the cardiac tissue. Rotation in the opposite direction removes the helix from the tissue to allow for repositioning of the lead at another location.

Combination devices are available for treating cardiac arrhythmias that are capable of delivering shock therapy for cardioverting or defibrillating the heart in addition to cardiac pacing. Such a device, commonly known as an implantable cardioverter defibrillator or "ICD", uses coil electrodes for delivering high-voltage shock therapies. An implantable cardiac lead used in combination with an ICD may be a quadrapolar lead equipped with a tip electrode, a ring electrode, and two coil electrodes. A quadrapolar lead requires four conductors extending the length of the lead body in order to provide electrical connection to each electrode.

Pacemaker systems, as well as other medical devices such as those mentioned above, can utilize a wide variety of lead designs. Many considerations are taken into account when optimizing the design of a lead. For example, minimizing lead size is important since a smaller device is more readily implanted within the cardiac structures or coronary vessels of a patient. Electrical insulation between multiple conductors and their associated electrodes is crucial to providing the desired therapeutic effect of electrical stimulation. Ideally, this insulation must be biostable or have predictable performance for the life of the lead, with minimal degradation of mechanical and electrical properties. With the increased number of insulated conductors required in quadrapolar and other multipolar leads, the diameter of the lead body is increased. It is desirable, however, to minimize the lead body diameter while maintaining proper insulation and the structural integrity of the lead.

Moreover, providing features that make a lead easier to implant and extract allows the clinician to complete the associated surgical procedure more safely and in less time. Finally, an optimized lead design is ideally manufactured using techniques that are relatively simple and easy to verify. The resulting product should be easy to test so that manufacturing defects can be detected prior to the implant of the device within a patient. What is needed, therefore, is an improved lead design that takes all of the foregoing factors into account, thereby providing a miniaturized implantable medical device configuration with improved long-term survival within the patient and providing suitable current carrying capacity for conducting pacing pulse or cardioversion/defibrillation shock therapy.

US 5,849,031 discloses a method for forming a conductor insulation within a medical lead of an implantable medical device comprising the steps of immersing a portion of a conductor wire within an anodization solution for producing an insulating oxide layer thereon, applying a bias voltage for a predetermined period of time, removing the bias voltage and advancing the conductor wire through a water rinse.

The present invention is a method for forming a conductor insulation within a medical device lead of an implantable medical device as defined in claim 1.

Such an implantable medical device could include a housing, having a connector block, generating electrical signals for delivering cardiac therapy, and a lead having a lead body extending from a proximal end to a distal end, the proximal end of the lead being insertable within the connector block and electrically coupling the housing and the lead. A plurality of conductor wires electrically couple the proximal end and the distal end of the lead body, with one or more of the plurality of conductor wires being formed of a material having a chemically modifiable surface for forming an insulating oxide layer thereon. An insulation layer, corresponding to the insulating oxide layer, is formed about the one or more of the plurality of conductor wires.

One or more of the plurality of conductor wires are formed of one of a refractory metal, an alloy of two or more refractory metals, and a semiconductor material. For example, the one or more of the plurality of conductor wires are formed of one of a tantalum metal and a tantalum-tungsten alloy, and the first insulation layer is an oxide of one of tantalum, tantalum-tungsten and other refractory metal or refractory metal alloy formed about the surface of the plurality of conductor wires. Or still further, the one or more of the plurality of conductor wires may be formed of one of a silicon-silicon dioxide, carbon fibers having a surface modified to diamond layers, a nitride, a carbon alloy, and a zirconium-zirconia composite.

The one or more of the plurality of conductor wires includes an inner layer having a surface, and an outer layer clad to the surface of the inner layer, with the inner layer being a high strength material having increased fatigue resistance and the outer layer corresponds to the chemically modifiable surface. The inner layer is preferably one of a refractory metal alloy and a semiconductor material, and the outer layer is a pure refractory metal. For example, the inner layer is tantalum-tungsten and the outer layer is one of tantalum and niobium.

The one or more of the plurality of conductor wires includes a core formed of a high conductivity material, such as gold, silver or molybdenum, for example.

The one or more of the plurality of conductor wires includes an inner layer having a surface, and an outer layer clad to the surface of the inner layer, with the inner layer being a high strength material having increased fatigue resistance and the outer layer corresponds to the chemically modifiable surface. In addition, the one or more of the plurality of conductor wires further includes a core formed of a high conductivity material such as gold, silver or molybdenum, for example. The inner layer is preferably one of a refractory metal alloy and a semiconductor material, and the outer layer is a pure refractory metal. For example, the inner layer is tantalum-tungsten and the outer layer is one of tantalum and niobium.

According to the present invention, a conductor insulation is formed within a medical device lead of an implantable medical device by immersing a first portion of a conductor wire formed of a material having a chemically modifiable surface for producing an insulating oxide layer thereon within an anodization solution corresponding to the producing of the insulating oxide layer. A bias is applied corresponding to a predetermined current limit and a corresponding voltage limit to the conductor wire, and a determination is made as to whether a predetermined cell anodization voltage has been reached. The predetermined cell anodization voltage is maintained for a predetermined time period, and the bias is removed and the conductor wire is advanced through a water rinse whereby a next portion of the conductor wire is immersed within the anodization solution.
FIG. 1 is a side plan view of an implantable medical device lead.
FIG. 2 is a cross-sectional view of a lead of an implantable medical device taken along cross-sectional lines II-II of FIG. 1.
FIG. 3 is a cross-sectional view of a lead of an implantable medical device taken along cross-sectional lines III-III of FIG. 1.
FIG. 4A is a cross-sectional view of a coiled wire conductor forming a multi-filar conductor coil.
FIG. 4B is a cross-sectional view of another coiled wire conductor forming a multi-filar conductor coil.
FIG. 5A is a cross-sectional view of another coiled wire conductor forming a multi-filar conductor coil.
FIG. 5B is a cross-sectional view of another coiled wire conductor forming a multi-filar conductor coil.
FIG 6A is a cross-sectional view of another coiled wire conductor forming a multi-filar conductor coil.
FIG 6B is a cross-sectional view of another coiled wire conductor forming a multi-filar conductor coil.
FIG 7A is a cross-sectional view of another coiled wire conductor forming a multi-filar conductor coil.
FIG 7B is a cross-sectional view of another coiled wire conductor forming a multi-filar conductor coil.
FIG. 8 is a schematic diagram of a conductor wire anodization system for forming a conductor insulation.
FIG. 9 is a flowchart of a method for forming conductor insulation in a medical device lead of an implantable medical device according to the present invention.

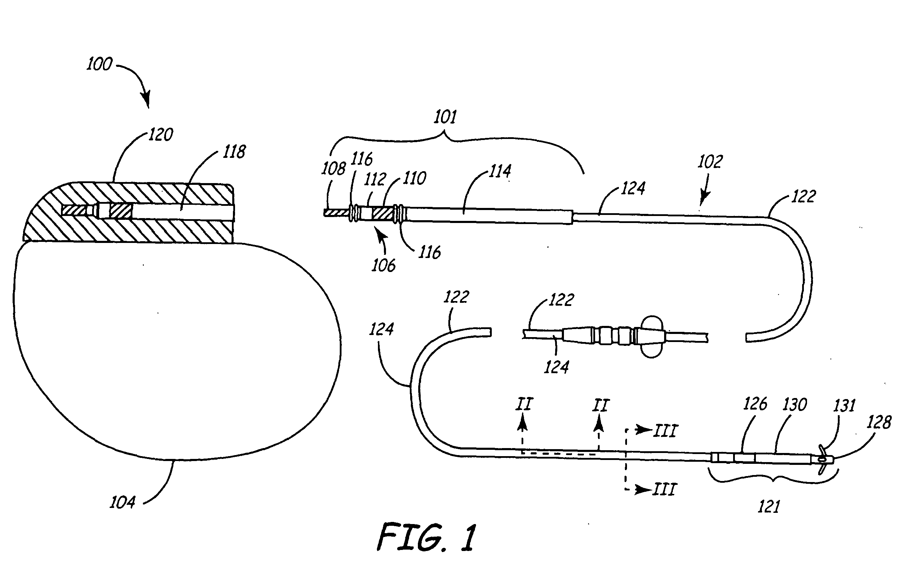

FIG. 1 is a schematic diagram of an implantable medical device. As illustrated in FIG. 1, the implantable medical device 100 includes an implantable medical device lead 102 and an implantable medical device housing 104, such as an implantable cardioverter/defibrillator or pacemaker/cardioverter/defibrillator (PCD), for processing cardiac data sensed through lead 102 and generating electrical signals in response to the sensed cardiac data for the provision of cardiac pacing, cardioversion and defibrillation therapies. A connector assembly 106 located at a proximal end 101 of lead 102 includes a conductive connector pin 108, a conductive ring element 110, and two insulative segments 112 and 114. Insulative segments 112 and 114 are each provided with a plurality of sealing rings 116 for sealing the proximally disposed connector pin 108 and ring element 110 within an elongated socket 118 located in a connector block 120 of housing 104 when connector assembly 106 is inserted within elongated socket 118 of conductor block 120 to electrically couple lead 102 with electronic circuitry (not shown) of housing 104.

Lead 102 includes an elongated lead body 122 that extends between proximal end 101 and a distal end 121 of lead 102. An outer insulative sheath 124 surrounds lead body 122 and is preferably fabricated of polyurethane, silicone rubber, or an ethylene tetrafluoroethylene (ETFE) or a polytetrafluoroethylene (PTFE) type coating layer. Coiled wire conductors are positioned within lead body 122, as will be described in detail below. Distal end 121 of lead 102 includes a proximal ring electrode 126 and a distal tip electrode 128, separated by an insulative sleeve 130. Proximal ring electrode 126 and distal tip electrode 128 are electrically coupled to conductive ring element 110 and conductive connector pin 108, respectively, by at least one coiled and/or cabled wire conductor in a manner shown, for example, in U.S. Patent Nos. 4,922,607 and 5,007,435.

Fixation of distal tip electrode 128 within the patient is assisted by an active or a passive fixation mechanism, which may or may not constitute a distal electrode, to maintain contact of distal tip electrode 128 with tissue to ensure adequate stimulation or sensing at distal end 121 of lead 102. For example, such fixation mechanism includes active, retractable and extendable helical coils (not shown) adapted to be extended and screwed into tissue at the desired site, or passive, soft pliant tines 131, as described, for example, in U.S. Patent No. 3,902,501, typically formed of silicone rubber or polyurethane.

Such pace/sense distal tip electrodes and fixation mechanisms are also currently used in conjunction with large surface area cardioversion/defibrillation electrodes extending proximally along the length of lead body 122 for either right atrial or ventricular placement. Separate electrical conductors and connectors are employed to connect the cardioversion/defibrillation electrodes with connector block 120 of housing 104 for applying cardioversion/defibrillation shock energy to the respective heart chamber. In this variation, electrode 126 may be considered to represent an elongated cardioversion/defibrillation electrode of any of the well-known types.

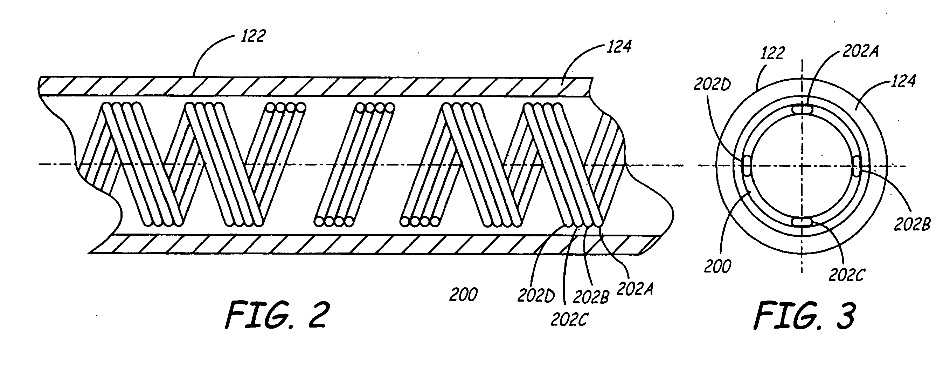

FIG. 2 is a cross-sectional view of a lead of an implantable medical device, taken along cross-sectional lines II-II of FIG. 1. As illustrated in FIG. 2, lead 102 of implantable medical device 100 includes a quadrifilar conductor coil 200 including four individual coiled wire conductors 202A, 202B, 202C and 202D extending within insulative sheath 124 of lead body 122. Coiled wire conductors 202A-202D electrically couple proximal ring electrode 126 and distal tip electrode 128 with conductive ring element 110 and conductive connector pin 108, respectively, of connector assembly 106.

FIG. 3 is a cross-sectional view of a lead of an implantable medical device, taken along cross-sectional lines III-III of FIG. 1. As illustrated in FIGS. 2 and 3, each of the individual coiled wire conductors 202A, 202B, 202C and 202D are parallel-wound in an interlaced manner to have a common outer and inner coil diameter. As a result, conductor coil 200 forms an internal lumen 204, which allows for passage of a stylet or guide wire (not shown) within lead 102 to direct insertion of lead 102 within the patient.

Alternately, lumen 204 may house an insulative fiber, such as ultrahigh molecular weight polyethylene (UHMWPE), liquid crystal polymer (LCP) and so forth, or an insulated cable in order to allow incorporation of an additional conductive circuit and/or structural member to aid in chronic removal of lead 102 using traction forces. Such an alternative would require insertion and delivery of lead 102 to a final implant location using alternate means, such as a catheter, for example. Lumen 204 may also include an insulative liner (not shown), such as a fluoropolymer, polyimide, PEEK, for example, to prevent damage caused from insertion of a style/guidewire (not shown) through lumen 204.

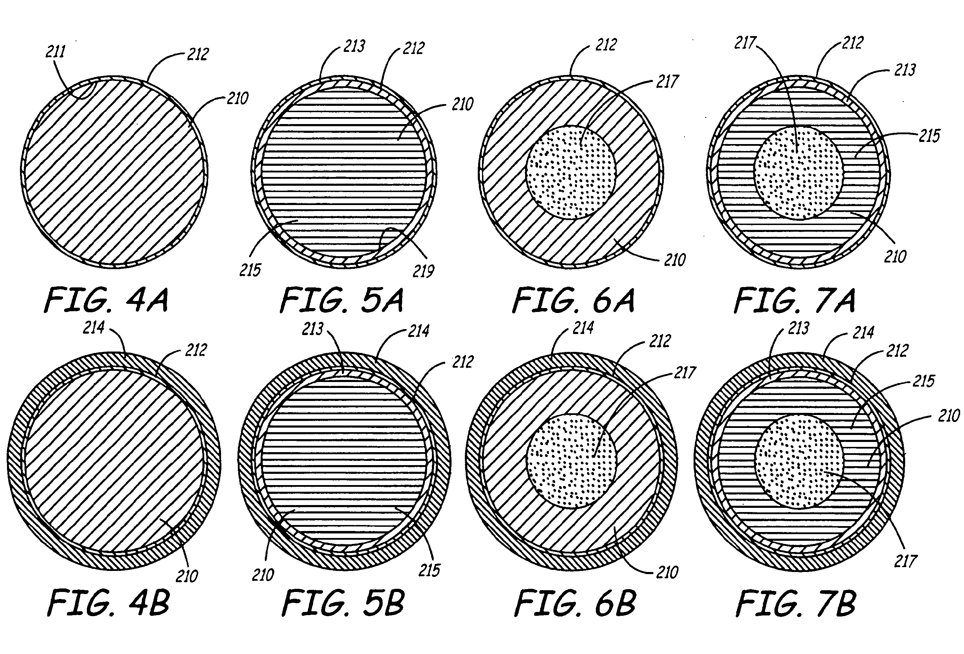

FIG. 4A is a cross-sectional view of a coiled wire conductor forming a multi-filar conductor coil. As illustrated in FIG. 4A, one or more of the individual coiled wire conductors 202A, 202B, 202C and 202D includes a conductor wire 210 surrounded by an insulative layer 212. The conductor wire 210 is a material having a surface that can be modified chemically (i.e., nanoengineered, anodized, etc.) or physically (chemical vapor deposition, physical vapor deposition, ion implantation, thermal oxidation, electric arc processing, or other process) to produce a native oxide, such as tantulum, tantalum-tungsten, or other refractory metal or alloy of refractory metals, or a semiconductor material, such as silicon-silicon dioxide, carbon fibers with a surface modified to diamond like or diamond layers, nitrides, carbide alloys, zirconium-zirconia composites, for example, so that insulative layer 212 is formed about conductor wire 210 using a process, described in detail below, in which a thin, flexible high dielectric oxide film is formed along a surface 211 of conductor wire 210.

For example, in order to maximize the quality of the oxide layer formed on conductor wire 210 that is produced to form insulative layer 212, conductor wire 210 is formed of tantalum and insulative layer 212 is a thin, flexible, high dielectric tantalum oxide film that is formed on tantalum conductive wire 210 using the process described below. In this way, by utilizing a material having a surface that can be modified chemically to produce a native oxide, the present invention reduces the thickness of lead 102, and improves metal ion induced oxidation resistance and radi-opacity of lead 102, compared with leads using less radi-opaque conductor materials, such as MP35N for example. Although conductor wire 210 is described in FIG. 4A as being formed of tantalum, it is understood that other materials having a surface that can be modified chemically to produce a native oxide, such as niobium, for example, may be utilized, and that therefore the present invention is not intended to be limited to the use of tantalum when maximizing the quality of the oxide layer formed on conductor wire 210 that is produced to form insulative layer 212.

In addition, in instances where maximizing the quality of the oxide layer formed on conductor wire 210 that forms insulative layer 212 is not a primary goal, it is understood that conductor wire 210 may be formed by a refractory metal alloy or semiconductor material, such as tantalum-tungsten, for example, so that insulative layer 212 is a thin, flexible, high dielectric oxide film that is formed on conductive wire 210 using the process described below.

FIG. 4B is a cross-sectional view of a coiled wire conductor forming a multi-filar conductor coil. As illustrated in FIG. 4B, one or more of coiled wire conductors 202A-202D of FIG 4A, described above, may include a redundant second insulative layer 214 positioned over conductor wire 210 and insulative layer 212, so that both insulative layer 212 and insulative layer 214 correspondingly electrically isolate each of coiled wire conductors 202A, 202B, 202C and 202D. Insulative layer 214 is preferably an ethylene tetrafluoroethylene (ETFE) or other fluoropolymer-type coating layer having high dielectric resistance, polyimide, or a combination of polyimide and ethylene tetrafluoroethylene (ETFE) or other fluoropolymer-type coating layers.

FIG. 5A is a cross-sectional view of a coiled wire conductor forming a multi-filar conductor coil. As illustrated in FIG. 5A, in order to increase the mechanical strength and the flex fatigue performance of lead 102, one or more of coiled wire conductors 202A, 202B, 202C or 202D is formed with conductor wire 210 being formed by an inner layer or layers 215 that is a refractory metal alloy or semiconductor material, such as tantalum tungsten, for example. A surface 219 of inner layer or layers 215 is clad with an outer layer 213 that is a material having optimal oxide formation, for instance pure tantalum or other pure refractory metal, such as niobium, for example. In addition, similar to what is described in FIG. 4A, insulative layer 212 is a thin, flexible, high dielectric oxide film that is formed on outer layer 213, so that, for example, if outer layer 213 is chosen to be tantalum, insulative layer 212 is a thin, flexible, high dielectric tantalum oxide film that is formed on outer layer 213 using the process described below.

In this way, by including inner layer or layers 215 that are composed of higher strength materials with increased flex fatigue resistance, the alternative of FIG. 5A enables the mechanical strength and flex fatigue performance of lead 201 to be increased when compared to lead 102 having conductor wire 210 as described above in reference to FIG. 4A. In addition, by including outer layer 213 with optimal oxide formation qualities, the oxide quality for forming the oxide layer resulting in insulative layer 212 is maintained.

FIG. 5B is a cross-sectional view of a coiled wire conductor forming a multi-filar conductor coil. As illustrated in FIG. 5B, one or more coiled wire conductors 202A-202D of FIG 5A, described above, may include redundant second insulative layer 214 positioned over outer layer 213 and inner layer 215, which form conductor wire 210, and insulative layer 212, so that both insulative layer 212 and insulative layer 214 correspondingly electrically isolate each of coiled wire conductors 202A, 202B, 202C and 202D. As described above, insulative layer 214 is preferably an ethylene tetrafluoroethylene (ETFE) or other fluoropolymer-type coating layer having high dielectric resistance, polyimide, or a combination of polyimide and ethylene tetrafluoroethylene (ETFE) or other fluoropolymer-type coating layers.

FIG 6A is a cross-sectional view of a coiled wire conductor forming a multi-filar conductor coil. As illustrated in FIG. 6A, in order to provide lead 102 according to the embodiment described above in FIG. 4A with increased conductive characteristics, one or more of the individual coiled wire conductors 202A-202D is formed as described above in reference to FIG. 4A and further includes a core 217 formed of a high conductivity material and centrally located within conductor wire 210, with conductor wire 210 being surrounded by insulative layer 212, as described above in reference to FIG. 4A above. The core 217 is formed of a material having high conductivity characteristics, such as gold, silver or molybdenum, for example.

In this way, by including core 217 within conductor wire 210, that is composed of a material having high conductivity characteristics, the alternate embodiment of FIG. 6A enables the conductive characteristics of lead 201 to be increased when compared to lead 102 that includes conductor wire 210 as described above in reference to FIG. 4A, while maintaining the oxide quality of the oxide layer resulting in insulative layer 212.

FIG 6B is a cross-sectional view of a coiled wire conductor forming a multi-filar conductor coil. As illustrated in FIG. 6B, one or more coiled wire conductors 202A-202D of FIG 6A, described above, may include redundant second insulative layer 214 positioned over outer layer 213 and inner layer 215, which form conductor wire 210, and insulative layer 212, so that both insulative layer 212 and insulative layer 214 correspondingly electrically isolate each of coiled wire conductors 202A, 202B, 202C and 202D. As described above, insulative layer 214 is preferably an ethylene tetrafluoroethylene (ETFE) or other fluoropolymer-type coating layer having high dielectric resistance, polyimide, or a combination of polyimide and ethylene tetrafluoroethylene (ETFE) or other fluoropolymer-type coating layers.

FIG 7A is a cross-sectional view of a coiled wire conductor forming a multi-filar conductor coil. As illustrated in FIG. 7A, in order to provide lead 102 according to the embodiment described above in FIG. 5A with increased conductive characteristics, one or more of the individual coiled wire conductors 202A-202D is formed as described above in reference to FIG. 5A and further includes core 217 formed of a high conductivity material and centrally located within conductor wire 210, with conductor wire 210 being surrounded by insulative layer 212, as described above in reference to FIG. 4A above. As previously described above, core 217 is formed of a material having high conductivity characteristics, such as gold, silver or molybdenum, for example.

In this way, by including inner layer or layers 215 that are composed of higher strength materials with increased flex fatigue resistance, along with outer layer 213 having optimal oxide formation qualities, and core 217 having high conductivity characteristics, the alternative of FIG. 7A increases both the mechanical strength and flex fatigue performance of lead 102 and the conductive characteristics of lead 102 when compared to lead 102 that includes conductor wire 210 as described above in reference to FIG. 4A, while maintaining the oxide quality of the oxide layer resulting in insulative layer 212.

FIG 7B is a cross-sectional view of a coiled wire conductor forming a multi-filar conductor coil. As illustrated in FIG. 7B, one or more coiled wire conductors 202A-202D of FIG 7A, described above, may include redundant second insulative layer 214 positioned over outer layer 213, inner layer 215 and core 217, which form conductor wire 210, and insulative layer 212, so that both insulative layer 212 and insulative layer 214 correspondingly electrically isolate each of coiled wire conductors 202A, 202B, 202C and 202D. As described above, insulative layer 214 is preferably an ethylene tetrafluoroethylene (ETFE) or other fluoropolymer-type coating layer having high dielectric resistance, polyimide, or a combination of polyimide and ethylene tetrafluoroethylene (ETFE) or other fluoropolymer-type coating layers.

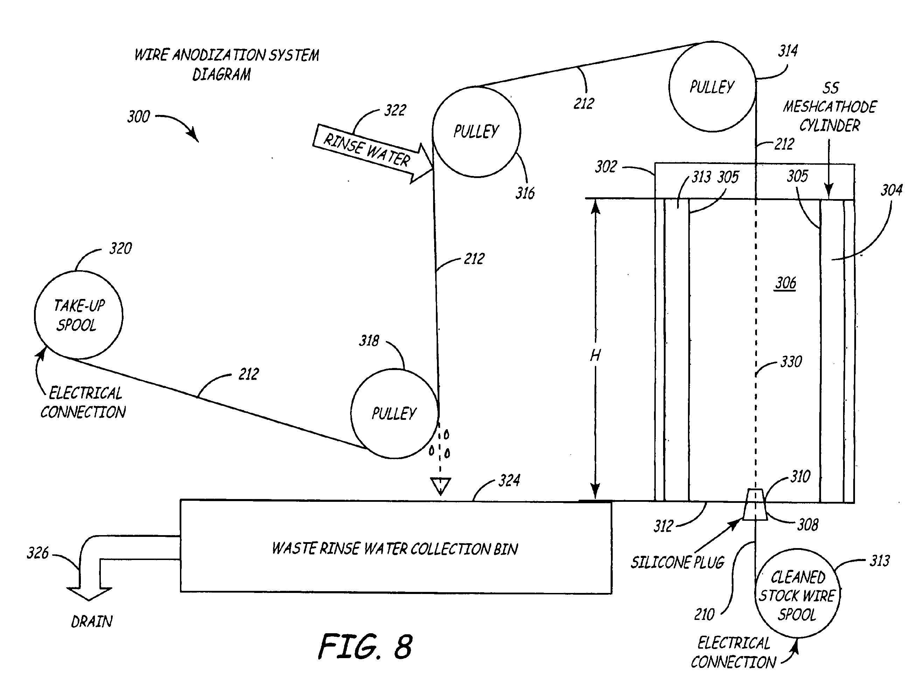

FIG. 8 is a schematic diagram of a conductor wire anodization system for forming a conductor insulation in a method according to the present invention. As illustrated in FIG. 8, a conductor wire anodization system 300 for forming insulative layer 212 on conductor wire 210, as described above, includes an anodization cylinder 302 and a stainless steel mesh cathode cylinder 304 inserted within anodization cylinder 302, along with an anodization solution 306, corresponding to the desired insulative layer 212, located within anodization cylinder 302 and cathode cylinder 304. Anodization solution 306 may be dilute sulfuric acid, phosphoric acid, or ammonium tartrate, for example. The anodization solution 306 is a dilute sulfuric acid at 1% volume. It is understood that anodization cylinder 302 and cathode cylinder 304 are shown cross-sectionally in FIG. 8. The cathode cylinder 304 extends a height H from a bottom portion 312 to a top portion 313, and anodization cylinder 302 is initially filled and maintained with anodization solution 306 so that anodization solution 306 remains at height H within anodization cylinder 302. As a result, anodization solution 306 remains at the same height H within anodization cylinder 302 as cathode cylinder 304 throughout the anodization of conductor wire 210 to form insulative layer 212. Height H of cathode cylinder 304 is not critical and can be chosen to correspond to any particularly relevant value. The anodization solution 306 is dilute sulfuric acid at approximately 1% volume, however, it is understood that anodization solution 306 is not intended to be limited to dilute sulfuric acid at 1% volume, but rather could include other volumes and/or solutions, such as phosphoric acid, or ammonium tartrate, for example.

The conductor wire 210 is a material having a surface that is chemically modifiable to produce a native oxide, such as tantalum and tantalum alloys, niobium and niobium alloys, and other refractory metals and refractory metal alloys and their oxides. As described above, conductor wire 210 may include inner layer or layers 215 and outer layer 213 and/or core 211 materials with different, more desirable properties, such as silver, gold, etc. for lower electrical resistivity, and/or TaW or other materials for higher strength and/or flex fatigue resistance. In this way, by utilizing a pure material as outermost surface 213, the present invention provides improved produceability and an optimized oxide growth to produce insulative layer 212.

A silicone plug 308 is positioned about conductor wire 210 to maintain a good moving seal between conductor wire 210 and plug 308. Once moving seal between plug 308 and conductor 212 is formed, plug is press-fit within a hole 310 formed at a bottom portion 312 of anodization cylinder 302 to form a press-fit seal between plug 308 and bottom portion 312 of anodization cylinder 302, and conductor wire 210 is routed from a spool 313 through conductor wire anodization system 300 over pulleys 314-318 and onto a take-up spool 320.

Pulley 314 is positioned over anodization cylinder 302, conductor wire 210 is positioned within plug 308, and hole 310 is centrally positioned along bottom portion 312 of anodization cylinder 302 so that conductor wire 210 is centrally located within anodization cylinder 302 and cathode cylinder 304 so that all sides of conductor wire 210 are equally spaced from an inner wall 305 of cathode cylinder 304.

A continuous water rinse 322 is applied to conductor wire 210 as conductor wire 210 passes between pulley 316 and 318 and excess water from water rinse 322 is collected in a water collection bin 324 and discarded through a drain 326.

Alternatively, the system features for carrying out the method of the present invention could be incorporated into a continuous spool-to-spool system; in this case, rather than anodizing discrete lengths of wire, a steady-state process is used, in which anodization of wire is done continuously as wire moves through the system spool-to-spool. If separate steps, which require different anodization parameters are necessary, multiple separate stages could be incorporated in this type of continuous process, with each stage accomplishing the various different process functions.

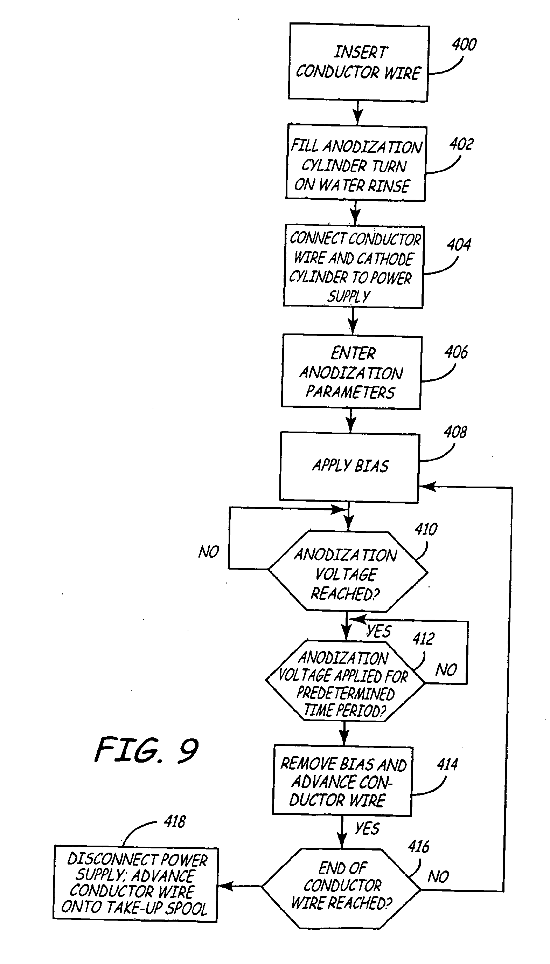

FIG. 9 is a flowchart of a method for forming conductor insulation in a medical device lead of an implantable medical device according to the present invention. As illustrated in FIGS. 8 and 9, according to the present invention, insulative layer 212 is formed on conductor wire 210 using conductor wire anodization system 300. Conductor wire 210 is inserted in conductor wire anodization system 300, Step 400, by first forming a moving seal between plug 308 and conductor wire 210. For example, silicone plug 308 is glued onto conductor wire 210, and subsequently cured broken free from conductor wire 210 to maintain a good moving seal between plug 308 and conductive wire 210. Plug 308 is then press-fit within hole 310 to form a press-fit seal between plug 308 and bottom portion 312 of anodization cylinder 302. Alternately, an o-ring seal mechanism, similar to that used in hemostasis valves, could be used to seal conductor wire 210. Conductor wire 210 is then routed from spool 313 and through conductor wire anodization system 300 by being advanced through plug 308, over pulleys 314-318 and onto take-up spool 320.

Once conductor wire 210 is routed from spool 313 to take-up spool 320, anodization cylinder 302 is then filled with anodization solution 306 and water rinse 322 is turned on, Step 402. Once a first end of conductor wire 210 along spool 313 and a second end of conductor wire 210 along take-up spool 320 are connected to a positive terminal of a power supply, and cathode cylinder 304 is connected to a negative terminal of the power supply, Step 404, anodization parameters, such as a desired current and voltage limitation, along with a corresponding time limit for maintaining a desired anodization voltage to form insulative layer 212, are entered into the power supply, Step 406. For example, according to a preferred embodiment of the present invention, it may be desirable to have a constant current limit within a range of approximately 0.5 to 2.5 mA/cm² and allow the cell voltage to reach approximately 50 volts to 200 volts for optimum properties, and to hold the maximum cell voltage, when reached, for between approximately 2-35 minutes, for example.

Conversely, the insulative layer 212 is produced by anodizing conductor wire 210 at a constant voltage rather than a constant current. Alternate embodiments can include a combination of constant current, constant voltage, pulse trains and any other suitable electrochemical method of forming an oxide.

Once the anodization parameters have been entered, the corresponding bias is applied to conductor wire 210 and cathode cylinder 304, Step 408, initiating the formation of an oxide layer along a portion 330 of conductor wire 210 extending within anodization solution 306 between plug 308 and top portion 313 of cathode cylinder 304.

Once the bias is applied to conductor wire 212, Step 408, a determination is made as to whether the desired anodization cell voltage has been reached, i.e., 50 volts for example, Step 410. In this way, by applying the current bias to conductor wire 210 and cathode cylinder 304, the anodization voltage is increased while a steady current is maintained until the predetermined anodization voltage limit is reached. Once the predetermined anodization voltage limit is reached, YES in Step 410, a determination is made as to whether the anodization voltage has been held steady for a predetermined time period, i.e., approximately 15 to 30 seconds, Step 412. In this way, once the predetermined anodization voltage limit has been reached and the anodization voltage has been applied for the predetermined time period, YES in Step 412, the bias is removed and conductor wire 210 is advanced from spool 313, along pulleys 314-318, through water rinse 322 and onto take-up spool 320, Step 414, so that a new portion 330 of conductor wire 210 extends within anodization solution 306 between plug 308 and top portion 313 of cathode cylinder 304.

Conductor wire 210 is advanced in Step 414 so that portion 313 of conductor wire 210 within anodization solution 306 between plug 308 and top portion 313 of cathode cylinder 304 is removed from cathode cylinder 304, and a new portion of conductor wire 210 is inserted within anodization solution 306 between plug 308 and top portion 313 of cathode cylinder 304. It is desirable to overlap portion 313 of conductor wire 210 that is removed from anodization solution 306 with the new portion of conductor wire 210 is inserted within anodization solution 306 to prevent the formation of gaps in insulative layer 212.

Once conductor wire 210 is advanced, a determination is made as to whether the end of conductor wire 210 within spool 313 has been reached, Step 416. For example, the end of conductor wire 210 is determined to be reached in Step 416 by monitoring the tension on conductor wire 210 and determining that the end of conductor wire 210 has been reached in response to increased tension, or in response to decreased tension, indicating conductor wire 210 has left spool 313. In addition, this monitoring of conductor wire 210 tension could also be utilized to detect other problems, such as conductor wire 210 sticking to adjacent wire-wraps on spool 313 (increased tension), or loss of seal provided by silicone sealings on conductor wire 210 (decreased tension). In the alternative, for example, the end of conductor wire 210 is determined to be reached by including an indicator tape or a wire length counter based on rotation of spool 313.

If the end of conductor wire 210 within spool 313 has not been reached, NO in Step 416, the bias is applied to conductor wire 212, Step 408, and Steps 408-412 are repeated for the new portion of conductor wire 210 advanced within. Once it is determined that the end of conductor wire 210 within spool 313 has been reached, YES in Step 416, the power supply is disconnected from conductor wire 210 and cathode cylinder 304, conductor wire 210 is advanced within take-up spool 320 so that all of conductor wire 210 is advanced through water rinse 322 and received within take-up spool 320, Step 418.

While particular embodiments of the method of the present invention have been shown and described, modifications may be made. For example, conductor wire 210 may be anodized to form insulative layer 212, covered with a much thinner polymer coating (which typically contain microdefects) and coiled or made into a cable. The resulting advantage is that since tantalum is corrosion resistance, it will be immune to pinhole corrosion. Another benefit of tantalum is that it will self heal if current leaks from any imperfection caused by handling of coiling, provided that the wire is in the positive polarity.

## Claims

1. A method for forming a conductor insulation within a medical device lead of an implantable medical device, comprising the steps of:
a) immersing a first portion of a conductor wire (210) formed of a material having a chemically modifiable surface for producing an insulating oxide layer thereon within an anodization solution (306) corresponding to the producing of the insulating oxide layer;
b) applying a bias corresponding to a predetermined current limit and a corresponding voltage limit to the conductor wire (210);
c) determining whether a predetermined cell anodization voltage has been reached;
d) maintaining the predetermined cell anodization voltage for a predetermined time period; and
e) removing the bias and advancing the conductor wire (210) through a water rinse (322) whereby a next portion of the conductor wire is immersed within the anodization solution (306).

2. The method of claim 1, wherein the first portion and the next portion of the conductor wire overlap.

3. The method of claim 1 or 2, further comprising the steps of:
determining whether an end of the conductor wire has been reached, and
repeating steps a-e.

4. The method of claim 1, 2 or 3, wherein step a) further comprises the steps of:
forming a moving seal between a plug and the conductor wire;
forming a press-fit seal between the plug and a bottom portion of an anodization cylinder containing the anodization solution;
routing the conductor wire through a conductor wire anodization system;
filling the anodization cylinder with the anodization solution to a height corresponding to a cathode cylinder inserted within the anodization cylinder;
electrically connecting the conductor wire and the cathode cylinder to a power supply; and
entering the predetermined current limit, the corresponding voltage limit, the predetermined anodization voltage, and the predetermined time period into the power supply.

5. The method of any of claims 1 to 4, wherein the conductor wire is formed of one of a refractory metal, an alloy of two or more refractory metals, and a semiconductor material and the anodization solution is a solution corresponding to the one of a refractory metal, an alloy of two or more refractory metals, and a semiconductor material.

## Patentansprüche

1. Verfahren zum Bilden einer Leiterisolierung in einer Medizinvorrichtungsleitung einer implantierbaren medizinischen Vorrichtung, umfassend die folgenden Schritte:
a) Eintauchen eines ersten Abschnitts eines Leiterdrahtes (210), der aus einem Material mit einer chemisch modifizierbaren Oberfläche zur Herstellung einer isolierenden Oxidschicht auf dieser gebildet ist, in eine dem Herstellen der isolierenden Oxidschicht entsprechende Anodisierungslösung (306);
b) Anlegen einer Vorspannung, die einem vorbestimmten Grenzstrom und einer entsprechenden Spannungsgrenze entspricht, auf den Leiterdraht (210);
c) Bestimmen, ob eine vorbestimmte Anodisierungszellspannung erreicht wurde;
d) Aufrechterhalten der vorbestimmten Anodisierungszellspannung über eine vorbestimmte Zeitdauer; und
e) Entfernen der Vorspannung und Vorwärtsbewegen des Leiterdrahtes (210) durch ein Wasserspülbad (322), wodurch ein nächster Abschnitt des Leiterdrahtes in die Anodisierungslösung (306) eingetaucht wird.

2. Verfahren nach Anspruch 1, wobei der erste Abschnitt und der nächste Abschnitt des Leiterdrahtes sich überschneiden.

3. Verfahren nach Anspruch 1 oder 2, ferner umfassend die folgenden Schritte:
Bestimmen, ob ein Ende des Leiterdrahtes erreicht wurde, und
Wiederholen der Schritte a) bis e).

4. Verfahren nach Anspruch 1, 2 oder 3, wobei Schritt a) ferner die folgenden Schritte umfasst:
Bilden einer beweglichen Abdichtung zwischen einem Stopfen und dem Leiterdraht;
Bilden einer Einpressabdichtung zwischen dem Stopfen und einem Bodenabschnitt eines die Anodisierungslösung enthaltenden Anodisierungszylinders;
Führen des Leiterdrahtes durch ein Leiterdraht-Anodisierungssystem;
Füllen des Anodisierungszylinders mit der Anodisierungslösung bis zu einer Höhe, die einem in den Anodisierungszylinder eingesetzten Kathodenzylinder entspricht; elektrisches Anschließen des Leiterdrahtes und des Kathodenzylinders an ein Netzgerät; und
Einstellen des vorbestimmten Grenzstromes, der entsprechenden Spannungsgrenze, der vorbestimmten Anodisierungsspannung und der vorbestimmten Zeitdauer an dem Netzgerät.

5. Verfahren nach einem der Ansprüche 1 bis 4, wobei der Leiterdraht aus einem der Folgenden gebildet wird: einem hitzebeständigen Metall, einer Legierung aus zwei oder mehr hitzebeständigen Metallen und einem Halbleitermaterial, und wobei die Anodisierungslösung eine Lösung ist, die jeweils dem hitzebeständigen Metall, der Legierung aus zwei oder mehr hitzebeständigen Metallen bzw. einem Halbleitermaterial entspricht.

## Revendications

1. Procédé de formation d'une isolation de conducteur à l'intérieur d'une dérivation de dispositif médical d'un dispositif médical implantable, comprenant les étapes consistant à :
a) immerger une première partie d'un fil conducteur (210), formé d'un matériau ayant une surface modifiable chimiquement pour produire une couche d'oxyde isolante sur celle-ci, à l'intérieur d'une solution d'anodisation (306) correspondant à la production de la couche d'oxyde isolante ;
b) appliquer une polarisation, correspondant à une limite de courant prédéterminée et une limite de tension correspondante, au fil conducteur (210) ;
c) déterminer si ou non une tension d'anodisation de cellule prédéterminée a été atteinte ;
d) maintenir la tension d'anodisation de cellule prédéterminée pendant une période de temps prédéterminée ; et
e) retirer la polarisation et faire avancer le fil conducteur (210) à travers un rinçage à l'eau (322), ce par quoi une partie suivante du fil conducteur est immergée à l'intérieur de la solution d'anodisation (306).

2. Procédé selon la revendication 1, dans lequel la première partie et la partie suivante du fil conducteur se chevauchent.

3. Procédé selon la revendication 1 ou la revendication 2, comprenant en outre les étapes consistant à :
- déterminer si ou non une extrémité du fil conducteur a été atteinte, et
- répéter les étapes a-e.

4. Procédé selon la revendication 1, 2 ou 3, dans lequel l'étape a) comprend en outre les étapes consistant à :
- former un joint d'étanchéité mobile entre une fiche et le fil conducteur ;
- former un joint d'étanchéité à ajustement serré entre la fiche et une partie inférieure d'un cylindre d'anodisation contenant la solution d'anodisation ;
- acheminer le fil conducteur à travers un système d'anodisation de fil conducteur ;
- remplir le cylindre d'anodisation de la solution d'anodisation jusqu'à une hauteur correspondant à un cylindre de cathode introduit à l'intérieur du cylindre d'anodisation ;
- connecter électriquement le fil conducteur et le cylindre de cathode à une alimentation électrique ; et
- entrer la limite de courant prédéterminée, la limite de tension correspondante, la tension d'anodisation prédéterminée et la période de temps prédéterminée dans l'alimentation électrique.

5. Procédé selon l'une quelconque des revendications 1 à 4, dans lequel le fil conducteur est formé de l'un d'un métal réfractaire, d'un alliage de deux métaux réfractaires ou davantage et d'un matériau semiconducteur et la solution d'anodisation est une solution correspondant à l'un d'un métal réfractaire, d'un alliage de deux métaux réfractaires ou davantage et d'un matériau semiconducteur.
